# EUROPEAN PATENT APPLICATION

(11) **EP 2 567 685 A2**
(43) Date of publication of application: **13.03.2013**
(21) Application number: 12005622.1
(22) Date of filing: 02.08.2012
(51) Int. Cl.: A61F 13/62, A44B 18/00

(54) **Multilayer female part of hook and loop fastener and method of producing it**

(30) Priority: 03.08.2011 IT BO20110484
(71) Applicant: MAGIS S.P.A., 50050 Cerreto Guidi (FI) (IT)
(72) Inventor: Marzi, Marco, 50053 Empoli (FI) (IT)
(74) Representative: Barberi, Vittorio

(57) **Abstract**

A multilayer material and a method for the formation of a multilayer material, usable material for the formation of anchorage area or "landing zones" for diapers and the like, of the type formed from a first layer or outer layer, which is provided with bushings and intended to constitute the "female" of a closing device fixable, and by a second layer or inner layer, which is intended to be attached to the diaper; the first layer (2) is formed by warp knit fabric and the second layer (3) is made of non-woven fabric.

## Description

The present invention relates to a multilayer material and a method for the manufacture of a multilayer material.

In particular, the multilayer material in question is used in the production of diapers and other articles for personal hygiene for the formation of the front part of the diaper, called in technical jargon "landing zone". The landing zone is the front part of the diaper that determines an attaching zone for resolvable closure means of the type called "Velcro" or "hook and loop", constituting the female part of the device, i.e. the part that supports the loops.

The material used for the formation of the landing areas is usually of multilayer type, with a top layer provided with loops to define the female part of the resolvable closing device.

In the most usual form, the landing zones are made with a multilayer material obtained by extrusion or glueing, with a process which can be relatively complex and relatively expensive if compared to the final cost of the product. In addition, the materials currently multilayer products can be relatively rigid, giving rise to the formation of a front part of the diaper which can be uncomfortable and / or not very suitable for the fit of the diaper. Some materials are characterized by a not optimal resistance to the repeated openings / closings.

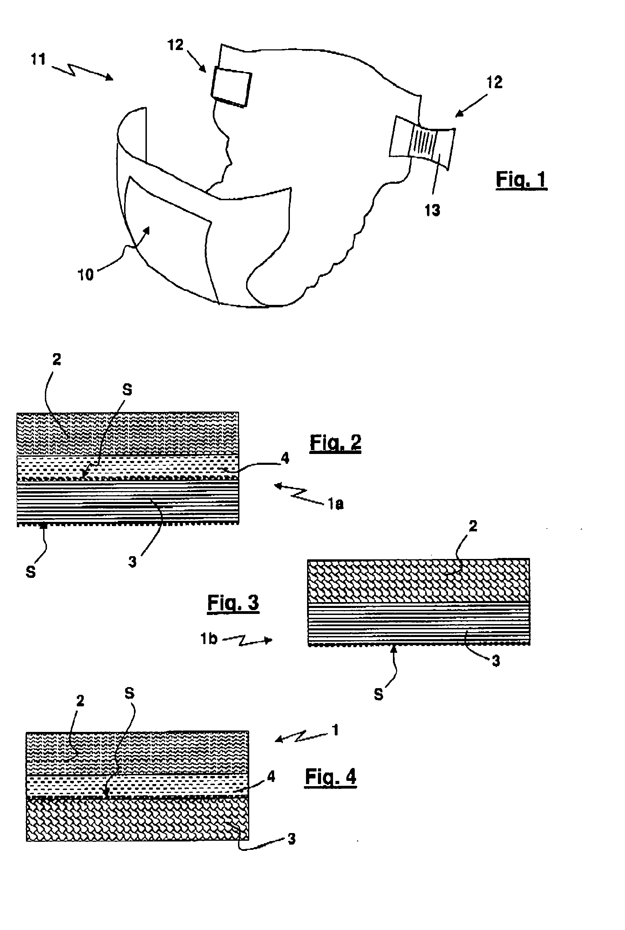

In Figs. 2 and 3 are shown in a schematic way two possible embodiments, of known type, of a multilayer material usable for the landing zone. In the drawings, a first layer is marked with 2, disposed upwardly, a second layer, disposed below, is marked with 3, and the possible layer of glue used to bond the two layers 2 and 3 is marked with 4. In conditions of use, when the multilayer material form the landing areas of a diaper, the first layer 2 is arranged externally, so as to present the loops outwards; the second layer 3 is disposed internally, permanently attached to the structure of the diaper .

In the example of Figure 2, the multilayer material 1a is formed by a first layer 2 in ladderproof or non-woven fabric, a second layer 3 in plastic film, the two layers being joined by a layer of glue 4. A disadvantage of this type of material is the limited softness (which determines less wearability and comfort of the article on which it is applied) and the not very appreciable aesthetical appearance.

In the drawings are identified with S the faces of the material that may be affected by the printing.

In the example of Figure 3 the multilayer material 1b is formed by a first layer 2 in non-woven fabric, a second layer 3 in plastic film, which is extruded and / or heat-sealed onto the layer of non-woven fabric to form the multi-layer material 1b. In this case, with respect to the solution previously described, the multilayer material 1b has mechanical characteristics generally not optimal as regards the resistance to the repeated opening and closing tests, due to a mechanical resistance of the fibers of the nonwoven fabric which is lower than that of the threads which constitute the warp-knit fabrics. Moreover, the obligation to print on the lower face (indicated with S) of the plastic film of the second layer 3, generally makes not very appreciable the printings made of such material. In fact, the printed design (formed on the lower face of the layer 3) is visible from the outside only in transparency, through the two layers 2 and 3.

An aim of the present invention is to provide a novel multilayer material and a method of making such material, capable of eliminating the drawbacks of the previous solutions and to provide at the same time a constructive methodology able to combine relatively low costs to a product of high quality. The multilayer material object of the invention is characterized by the fact of offering an extremely high softness, if compared to the materials of known type, suitable to give to the diaper a high wearability; the multilayer material has optimal mechanical characteristics, with a considerable resistance to the repeated openings / closings of the resolvable closing device; in addition, the material of the invention can be printed in a precise and effective way on the upper face. This result is achieved, according to the invention by adopting the idea of a material and a method having the characteristics described in the independent claims. Other features are the subject of the dependent claims.

Among the advantages of the present invention are the following:
- the material is very soft;
- the articles which have the landing zones made with the material of the invention are extremely comfortable, with a high wearability;
- the material has high mechanical properties with respect to repeated openings /closings;
- it is possible to print the multilayer material in an effective way, providing a pleasing aesthetic effect;
- the production costs are not higher than those of the known techniques, while offering a higher quality product.

These and other advantages and features of the present invention will be best understood by anyone skilled in the art from the following description and with the help of the attached drawings given as a practical exemplification of the invention, but not to be considered in a limiting sense, in which:
- Fig 1 is a schematic perspective view of a diaper provided with the "landing zone";
- Fig 2 is a schematic sectional view of a possible embodiment of a multilayer material of known type;
- Fig 3 is a schematic sectional view of another embodiment of a multilayer material of known type;
- Fig 4 is a schematic sectional view of a possible embodiment of a multilayer material made in accordance with the invention.

As previously said, the present invention relates to a multilayer material (1) usable in the production of diapers and other articles for personal hygiene for the formation of the front part said "landing zone". In Fig.1, the landing zone is marked with (10) and is arranged at the front part of the diaper (11). The landing area (10) forms the female part (provided with "loops") of a resolvable closure previously (12), provided with a respective male part (13), provided with "hooks".

In accordance with the invention, the material (1) used for the formation of the landing zones is of the multilayer type, with an upper layer (2) provided with loops to define the female part of the resolvable closing device.

The multilayer material according to the invention comprises:
- a first layer (2) formed from a warp-knit fabric (also called "knitted textile");
- a second layer (3) non-woven fabric;
- a layer of glue (4) interposed between said first (2) and second layer (3), which are bound together by the glue.

In particular, the first layer (2) may be a warp knit fabric of various kinds: polyamide, polyester, mixed (for example with polyamide weft and polyester warp or vice versa), based on biodegradable polymers (PLA), etc etc. Advantageously, the warp-knit fabric which forms the outer layer (2) may be of the type called in technical jargon as "standing loop", as that described in EP-1811070. In particular, the mesh and the loops of the first layer (2) can be realized in a single body, with the loops simultaneously present both in the longitudinal direction and transverse direction. In addition, the threads forming such a layer may be in monofilament synthetic yarns made from polyester, polyamide, polyamide and / or polypropylene, with or without aloe vera. The monofilament yarn has a range from 15 to 50 den. With this type of fabric, the loops provide a better grip for the hooks, even in the case of repeated openings /closings, as experimentally evidenced with proofs of detachment with dynamometer, both in the tests of "peel" that of "shear". In addition, (and especially considering that the layer 3 is a non-woven fabric having therefore generally more irregular surfaces compared to common plastic films) the operation of bonding to join the two layers (2) and (3) does not damage the same loops, which are normally raised (projected outwards) and, therefore, not affected by the action of the glue.

As expressed before, the second layer (3) is a non-woven fabric, the second layer (3) is of polyolefinic nature and / or with fibers made from biodegradable polymers; moreover, non-woven fabrics of spunbond type, spunlace or air-through bonds can be used, as well as mixed systems such as spunbond / spunlace etc etc. The experimental tests have shown the preferable quality of spunbond type, since it appeared more suitable to be joined for bonding to the structure of the diaper for its compactness presented by the fibers constituting the layer (3). The composition of the nonwoven fabric can be of various nature: polypropylene, polyester, mixed (for example with different percentages of polyester and polypropylene), with fibers made from biodegradable polymers (PLA), etc etc.

The glue (4) can be of various nature: a polyurethane (for example of the type "hot melt" or "solvent less"), hot-melt thermo-adhesive (for example, polyolefin-based or the type EVA), etc etc.

The particular arrangement of the layers of the multilayer material (1) of the present invention allows the printing on the upper face of the second layer (3) (as indicated by arrow S in Fig.4).

This feature allows to manufacture a product able to provide a technical and aesthetic effect of considerable importance. In practice, it is obtained a product with very high technical and aesthetic characteristics at a relatively low cost. Furthermore, it is advantageous the use of inks of the type UV-crosslinkable without solvents. In other words, the second layer (3) is printed on its upper face (that intended to be arranged externally) with UV-crosslinkable inks which, when subjected to irradiation with UV lamps are to be fixed by crosslinking, thanks the polymerization of the ink.

Advantageously, the materials used to form the multilayer material may be biodegradable and / or biocompatible; in this way, the disposal of diapers will be extremely compatible from the ecological point of view.

In accordance with the invention, a process for the formation of the multilayer material (1) is characterized by the fact of joining through the passage through a pair of rolls or calender rolls a first layer (2) formed by a warp-knit fabric and a second layer (3) in non-woven fabric, between said first and second layer being interposed a layer of a glue.

The materials usable for the first layer (2), for the second layer (3) and for the glue (4) have been previously indicated.

In accordance with the invention, also the process for the formation of multilayer material (1) provides to print on the upper face of the second layer (3) non-woven fabric: in this way the printing is clearly visible and is not damaged by the repeated operations of opening and closing because the engagement portion is formed only by the warp knit fabric that protects in this way the nonwoven fabric.

The construction details may vary in any equivalent way as in the shape, dimensions, elements disposition, nature of the materials used, without however departing from the scope of the adopted solution and thus remaining within the limits of the protection granted by this patent.

## Claims

1. Multilayer material usable for the formation of the anchorage area or "landing zone" for diapers and similar items, material comprising a first layer or outer layer, which is provided with loops and intended to constitute the "female" of a releasable closure device, and a second layer or inner layer, which is intended to be fixed to the diaper; material **characterized in that**:
- said first layer (2) is formed by warp-knit fabric, polyester and / or polyamide, polyamide and / or polypropylene, with or without aloe vera;
- said second layer (3) is made of non-woven fabric, of polyolefinic nature and /or with fibers made from biodegradable polymers; said second layer being of spunbond, spunlace or air-through bond type, or of mixed type composed by the previous types;
- the two layers are joined by gluing.

2. Material according to claim 1, **characterized in that** the first layer (2) is provided with loops of the type called "standing loop", i.e. of the type described in EP-1811070.

3. Material according to claim 2, **characterized in that** the meshes and the loops of the first layer (2) can be realized in a single body, with the loops simultaneously present both in the longitudinal direction and transverse direction.

4. Material according to claim 2, **characterized in that** the strands forming said first layer (2) are synthetic monofilament yarns made from polyester and / or polyamide, polyamide and / or polypropylene, with or without aloe vera, of range from 15 to 50 den.

5. Material according to claim 1, **characterized in that** the second layer (3) is a nonwoven fabric of spunbond type.

6. Material according to one of the preceding claims, **characterized in that** the second layer (3) is printed on its upper face (S), intended to be arranged outside in use.

7. Material according to claim 6, **characterized in that** the second layer (3) is printed with UV- crosslinkable ink.

8. Method for the formation of a multilayer material usable for the formation of the anchorage area or "landing zone" for diapers and similar items, material comprising a first layer or outer layer, which is provided with loops and intended to constitute the "female" of a releasable closure device, and a second layer or inner layer, which is intended to be fixed to the diaper; method **characterized in that** a first layer (2) formed from a warp-knit fabric and a second layer (3) nonwoven fabric are joined passing through a pair of rolls or calender rolls, between said first and second layer being interposed a layer of glue; said method in which:
- said first layer (2) is formed by warp-knit fabric, polyester and / or polyamide, polyamide and / or polypropylene, with or without aloe vera, and
- said second layer (3) is made of non-woven fabric, of polyolefinic nature and / or with fibers made from biodegradable polymers; said second layer being of type spunbond, spunlace or air-through bond, or of mixed type composed by the previous types.

9. Method according to claim 8, **characterized in that** the first layer (2) is of type with loops of the type called "standing loop", i.e. of the type described in EP-1811070

10. Method according to claim 9, **characterized in that** the meshes and the loops of the first layer (2) can be realized in a single body, with the loops simultaneously present both in the longitudinal direction and transverse direction.

11. Method according to claim 9, **characterized in that** the threads forming said first layer (2) are synthetic monofilament wires made from polyester and / or polyamide, polyamide and / or polypropylene, with or without aloe vera, with range from 15 to 50 den.

12. Method according to claim 8, **characterized in that** the second layer (3) is a non-woven type of spunbond, spunlace or air-through bond, or of mixed type composed by the previous types.

13. Method according to one of claims 8 to 12, **characterized in that** the second layer (3) is printed on its upper face (S), intended to be arranged outside in use.

14. Method according to claim 13, **characterized in** printing the second layer (3) with UV- crosslinkable ink and subjecting to irradiation with UV lamps.
